(19) Europäisches Patentamt
European Patent Office
Office européen des brevets

(11) EP 4 509 574 B1

(12) # EUROPEAN PATENT SPECIFICATION

(45) Date of publication and mention of the grant of the patent:
**22.04.2026 Bulletin 2026/17**

(21) Application number: **23788150.3**

(22) Date of filing: **24.03.2023**

(51) International Patent Classification (IPC):
***C09J 163/00*** *(2006.01)* ***A61L 31/10*** *(2006.01)*
***A61B 1/00*** *(2006.01)* ***C08G 59/24*** *(2006.01)*
***C08G 59/62*** *(2006.01)* ***C08G 59/68*** *(2006.01)*
***C08K 3/34*** *(2006.01)* ***C08K 3/36*** *(2006.01)*

(52) Cooperative Patent Classification (CPC):
(C-Sets available)
**A61L 31/10; A61B 1/00; C08G 59/245; C08G 59/621; C08G 59/686; C09J 161/04; C09J 163/00;** A61B 1/0011; C08K 2201/003; C08K 2201/011 (Cont.)

(86) International application number:
**PCT/JP2023/011954**

(87) International publication number:
**WO 2023/199730 (19.10.2023 Gazette 2023/42)**

(54) **ADHESIVE AGENT FOR MEDICAL DEVICES, CURED ARTICLE, MEDICAL DEVICE MEMBER, MEDICAL DEVICE, AND METHOD FOR MANUFACTURING MEDICAL DEVICE**

HAFTMITTEL FÜR MEDIZINISCHE VORRICHTUNGEN, GEHÄRTETER ARTIKEL, MEDIZINPRODUKTELEMENT, MEDIZINISCHE VORRICHTUNG UND VERFAHREN ZUR HERSTELLUNG DER MEDIZINISCHEN VORRICHTUNG

AGENT ADHÉSIF POUR DISPOSITIFS MÉDICAUX, ARTICLE DURCI, ÉLÉMENT DE DISPOSITIF MÉDICAL, DISPOSITIF MÉDICAL ET PROCÉDÉ DE FABRICATION DE DISPOSITIF MÉDICAL

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **14.04.2022 JP 2022066686**

(43) Date of publication of application:
**19.02.2025 Bulletin 2025/08**

(73) Proprietor: **FUJIFILM Corporation**
**Tokyo 106-8620 (JP)**

(72) Inventors:
- **FURUKAWA, Kazushi**
  **Ashigarakami-gun, Kanagawa 258-8577 (JP)**
- **NAKAI, Yoshihiro**
  **Ashigarakami-gun, Kanagawa 258-8577 (JP)**

(74) Representative: **HGF**
**HGF Limited**
**4th Floor, 1 City Square**
**Leeds LS1 2ES (GB)**

(56) References cited:
**WO-A1-2020/175277** **CN-A- 113 185 804**
**JP-A- 2011 026 457** **JP-A- 2019 041 873**
**JP-B2- 4 875 790** **US-A1- 2008 251 203**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C09J 163/00, C08K 3/36, C08K 3/34**

## Description

### BACKGROUND OF THE INVENTION

1. Field of the Invention

The present invention relates to an adhesive for a medical device, a cured substance, a medical device member, a medical device, and a manufacturing method of a medical device.

2. Description of the Related Art

Among adhesives, epoxy-based adhesives have excellent workability, and also exhibit excellent adhesiveness, electrical properties, heat resistance, moisture resistance, and the like in a case of being a cured substance. Therefore, the epoxy-based adhesive has been used in various fields and also studied for use in fixing a constituting member of a medical device. For example, JP2019-41873A discloses a two-liquid type adhesive for an endoscope, containing a main agent and a curing agent, in which the main agent includes at least one epoxy resin of a bisphenol A-type epoxy resin, a bisphenol F-type epoxy resin, or a phenol novolac-type epoxy resin, and the curing agent includes an imidazole compound. According to the technique disclosed in JP2019-41873A, the above-described main agent may contain a filler such as silica, and the above-described adhesive for an endoscope can maintain sufficient adhesive force even in a case where a cured substance obtained by a curing reaction is repeatedly subjected to a sterilization treatment by an autoclave, EOG, hydrogen peroxide plasma, or the like.

### SUMMARY OF THE INVENTION

The medical device is used repeatedly over a long period of time, and it is required that a medical device member or a medical device has a structure which is resistant to growth of bacteria even with repeated cleaning, in addition to durability for the sterilization treatment as described above. As a result of studying adhesives for a medical device in the related art, the present inventors have found that, in a case where a filler contained in the cured substance of the adhesive is unevenly distributed in the cured substance, moisture can infiltrate between the constituting member of the medical device and the cured substance, leading to growth of bacteria.

In addition, since some medical devices are inserted into the human body, it is required that the above-described cured substance has no color unevenness (color is substantially uniform) or the like to avoid causing discomfort to the subject. Furthermore, it is also required that the above-described cured substance maintains sufficient adhesive force for a long period of time while the constituting member is fixed, and that the constituting member is less likely to be damaged by cracks or the like even in a case of being subjected to an impact due to a collision between devices or the like (impact resistance). Furthermore, in order to obtain the medical device more quickly, it is required that the curing reaction is performed at a temperature considerably higher than room temperature (for example, 80°C) while satisfying the above-described requirements.

An object of the present invention is to provide an adhesive for a medical device and a cured substance thereof, which are suitable for fixing a constituting member of a medical device, in which the cured substance to be obtained has little color unevenness even in a case where a curing reaction is performed at a temperature considerably higher than room temperature (for example, 80°C), it is possible to prevent growth of bacteria on a medical device even in a case where the medical device is used for a long period of time while a constituting member of the medical device is fixed, and it is possible to maintain sufficient adhesive force between constituting members and suppress damage or the like caused by a collision between devices. Another object of the present invention is to provide a medical device member which is suitable as a constituting member of a medical device and includes the above-described cured substance, a medical device including the medical device member, and a manufacturing method of a medical device.

As a result of intensive studies, the present inventors have found that, in an epoxy-based adhesive, in a case where an imidazole compound having a specific structure and having a melting point or lower than 60°C is used in a specific amount as a curing component of a curing agent which acts on an epoxy resin, and a phenol resin which is liquid at 25°C and a specific inorganic filler (fumed silica and talc having a specific average particle diameter) are used in a specific amount, a cured substance obtained by the adhesive has little color unevenness, it is possible to prevent growth of bacteria on a medical device even in a case where the medical device is used for a long period of time while a constituting member of the medical device is fixed, and it is possible to maintain sufficient adhesive force between constituting members and suppress damage or the like caused by a collision between devices. The present invention has been completed by further repeating studies on the basis of the above-described finding.

The foregoing objects of the present invention have been achieved by the invention as defined in the claims.

In the present invention, numerical values denoted before and after are used to include the numerical values as a

lower limit value and an upper limit value.

With the adhesive for a medical device according to the aspect of the present invention, a cured substance to be obtained has little color unevenness even in a case where a curing reaction is performed at a temperature considerably higher than room temperature (for example, 80°C), it is possible to prevent growth of bacteria on a medical device even in a case where the medical device is used for a long period of time while a constituting member of the medical device is fixed, and it is possible to maintain sufficient adhesive force between constituting members and suppress damage or the like caused by a collision between devices. In addition, the cured substance according to the aspect of the present invention has little color unevenness, it is possible to prevent growth of bacteria on a medical device even in a case where the medical device is used for a long period of time while a constituting member of the medical device is fixed, and it is possible to maintain sufficient adhesive force between constituting members and suppress damage or the like caused by a collision between devices. In addition, the medical device member according to the aspect of the present invention includes the cured substance according to the aspect of the present invention in an adhesive portion of a constituting member, it is possible to prevent growth of bacteria on the medical device even in a case where the medical device is used for a long period of time, and it is possible to maintain sufficient adhesive force between constituting members and suppress damage or the like caused by a collision between devices, so that the medical device member is suitable for a constituting member of a medical device. Therefore, in the medical device according to the aspect of the present invention, which includes the above-described medical device member, it is possible to prevent growth of bacteria in a case of being used for a long period of time, and it is possible to maintain sufficient adhesive force between constituting members and suppress damage or the like caused by a collision between devices.

With the manufacturing method of a medical device according to the aspect of the present invention, it is possible to obtain the above-described medical device.

## BRIEF DESCRIPTION OF THE DRAWINGS

Fig. 1 is a cross-sectional view schematically showing an embodiment of the medical device member according to the present invention.

## DESCRIPTION OF THE PREFERRED EMBODIMENTS

[Adhesive for a medical device]

Preferred embodiments of the adhesive for a medical device according to the present invention will be described.

The adhesive for a medical device according to the embodiment of the present invention (hereinafter, also referred to as "adhesive according to the embodiment of the present invention") contains the following components (a) to (e).

(a) an epoxy resin including at least one of a bisphenol A-type epoxy resin or a bisphenol F-type epoxy resin
(b) an imidazole compound having no heteroatom other than a ring-constituting nitrogen atom and having a melting point of lower than 60°C
(c) a phenol resin which is liquid at 25°C
(d) fumed silica having an average particle diameter of 5 nm or more and 35 nm or less
(e) talc

The above-described epoxy resin (a) (hereinafter, also simply referred to as "component (a)") is a main agent of the adhesive, and the above-described imidazole compound (b) having no heteroatom other than a ring-constituting nitrogen atom and having a melting point of lower than 60°C (hereinafter, also simply referred to as "component (b)") is a curing component with reacts with the epoxy resin to cure the adhesive. The above-described phenol resin (C) which is liquid at 25°C (hereinafter, also simply referred to as "component (c)"), the above-described fumed silica (d) having an average particle diameter of 5 nm or more and 35 nm or less (hereinafter, also simply referred to as "component (d)"), and the above-described talc (e) (hereinafter, also simply referred to as "component (e)") have an effect of, for example, increasing a viscosity of a composition obtained by mixing (a) to (e) or reducing temperature dependence of the viscosity of the composition.

In the adhesive according to the embodiment of the present invention, with respect to 100 parts by mass of the total content of the above-described bisphenol A-type epoxy resin and the above-described bisphenol F-type epoxy resin, a content of the above-described component (b) is 1 to 20 parts by mass, a content of the above-described component (c) is 1 to 20 parts by mass, a content of the above-described component (d) is 1 to 20 parts by mass, a content of the above-described component (e) is 5 to 30 parts by mass, and the total of the content of the above-described component (d) and the content of the above-described component (e) is 6 parts by mass or more and less than 40 parts by mass.

A form of the adhesive according to the embodiment of the present invention is not particularly limited as long as it contains the above-described respective components. For example, the adhesive according to the embodiment of the present invention may contain a mixture of the above-described components (a) to (e) (one-liquid type), or may contain the components (a) to (e) in a state in which some components of the above-described components (a) to (e) are separated from the other components (two- to four-liquid type). In addition, the adhesive according to the embodiment of the present invention may contain the components (a) to (e) in a state in which each component is separated from the others (five-liquid type). Any of these forms are included in the adhesive according to the embodiment of the present invention.

In the present specification, in a case of describing the content of each component in the adhesive or specifying the content of each component in the adhesive in the present invention, in any form of the two- to five-liquid types, mixing of the components (a) to (e) during use means that the content of each component in the mixture satisfies the above-described desired content. That is, in any form of the two- to five-liquid types, in a case where the components are in a state of being separated from each other, it is not necessary for each content of the components (a) to (e) to satisfy the content described in the present specification or the content defined in the present invention. That is, in any form of the two-to five-liquid types, the content described in the present specification or the content defined in the present invention is satisfied immediately before a timing of mixing the components (a) to (e) during use.

In a case where the adhesive according to the embodiment of the present invention is the one-liquid type, or in a case where components which can react with each other are mixed in the two-liquid type or the like (for example, in a case where the epoxy resin and the curing component are mixed), in order to maintain each component in a stable state without causing a reaction between the components or to sufficiently suppress the reaction between the components, it is preferable that the above-described adhesive is stored at a low temperature at which the reaction does not substantially occur. For example, the adhesive can be stored at -20°C or lower, preferably -30°C or lower, more preferably -40°C or lower, and still more preferably -50°C or lower. In addition, the adhesive can be stored in a light-shielded manner as necessary.

With the adhesive for a medical device according to the embodiment of the present invention, a cured substance to be obtained has little color unevenness even in a case where a curing reaction is performed at a temperature considerably higher than room temperature (for example, 80°C), it is possible to prevent growth of bacteria on a medical device even in a case where the medical device is used for a long period of time while a constituting member of the medical device is fixed, and it is possible to maintain sufficient adhesive force between constituting members and suppress damage or the like caused by a collision between devices. The reason for this is not clear, but it is considered that the adhesive according to the embodiment of the present invention contains each component in a specific amount, so that a thickening effect of each of the components (c) to (e) can be expressed without inhibition. This allows a viscosity of the adhesive at around room temperature during application to be maintained to a certain extent even in a case of being cured at a high temperature, and effectively suppresses sedimentation of the components contained in the adhesive.

The adhesive according to the embodiment of the present invention is used mainly for fixing various members which constitute a medical device (constituting members of a medical device). That is, the adhesive according to the embodiment of the present invention is used for adhering a constituting member of a medical device to other constituting members of the medical device, thereby fixing the constituting member of the medical device to the other constituting members. The adhesive used for fixing the constituting member of the medical device is cured to be a cured substance and form an adhesive portion of the medical device.

A member to be fixed by the adhesive according to the embodiment of the present invention is not particularly limited, and examples thereof include a metal member, a glass member, and a resin member. The "fixing" of the constituting member of the medical device is performed by adhering the constituting member of the medical device to another component (support member) which constitutes the medical device. The support member may be a tube wall or the like of the medical device, a non-movable member fixed to the tube wall or the like, or a member in which a relative position in the medical device can be moved, such as a tube. In addition, the term "fixing" in the present invention is used to mean that a space between the constituting member of the medical device and the support member in which the constituting member of the medical device is incorporated is filled with the cured substance of the adhesive, that is, sealed.

In addition, the cured substance of the adhesive according to the embodiment of the present invention can also be used as the constituting member of the medical device itself.

Each component constituting the adhesive according to the embodiment of the present invention will be described below.

<(a) Epoxy resin>

The adhesive according to the embodiment of the present invention contains, as the component (a), an epoxy resin including at least one of a bisphenol A-type epoxy resin or a bisphenol F-type epoxy resin.

The adhesive according to the embodiment of the present invention may contain only one type of the component (a), or a combination of two or more types of the component (a).

Since the color unevenness of the cured substance according to the embodiment of the present invention can be effectively suppressed by an intermolecular interaction of a bisphenol structure included in the bisphenol A-type epoxy resin or the bisphenol F-type epoxy resin, the growth of bacteria on the medical device according to the embodiment of the present invention can be effectively prevented, and the damage due to the collision between devices or the like is less likely to occur, it is preferable that the above-described component (a) contains an epoxy resin represented by General Formula (1).

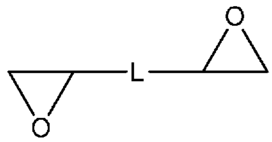 (1)

In General Formula (1), L represents a linking group formed by combining at least one of an alkylene group, an alkenylene group, or an alkynylene group with an ether bond, an alkylene group, an alkenylene group, or an alkynylene group.

L preferably represents a linking group formed by combining at least one of an alkylene group, an alkenylene group, or an alkynylene group with an ether bond, or an alkylene group; and more preferably represents a linking group formed by combining an alkylene group with an ether bond.

The alkylene group included in L may be linear, branched, or cyclic. The number of carbon atoms in the alkylene group preferably is 1 to 30, more preferably 1 to 20, and still more preferably 1 to 10.

The alkenylene group included in L may be linear, branched, or cyclic. The number of carbon atoms in the alkenylene group preferably is 2 to 30, more preferably 2 to 20, and still more preferably 2 to 10.

The alkynylene group included in L may be linear, branched, or cyclic. The number of carbon atoms in the alkynylene group preferably is 2 to 30, more preferably 2 to 20, and still more preferably 2 to 10.

L is preferably a combination of an alkylene group and an oxygen atom. Since the cured substance according to the embodiment of the present invention is hydrophobic and can suppress moisture absorption, the alkylene group preferably includes an alkylene group having 4 to 15 carbon atoms, more preferably includes an alkylene group having 4 to 10 carbon atoms, and still more preferably includes an alkylene group having 4 to 8.

The epoxy resin represented by General Formula (1) is preferably an alkanediol diglycidyl ether. The number of carbon atoms in the alkane is preferably 4 to 15, more preferably 4 to 10, and still more preferably 4 to 8. The alkane is preferably linear.

A chemical formula weight of L is not particularly limited, and for example, is preferably 30 to 400 and more preferably 50 to 150.

An epoxy equivalent of the epoxy resin contained in the adhesive according to the embodiment of the present invention is preferably 10 to 1,000, more preferably 50 to 500, still more preferably 80 to 400, and particularly preferably 100 to 300. The epoxy resin contained in the adhesive according to the embodiment of the present invention usually has two or more epoxy groups in one molecule.

The epoxy equivalent is a value obtained by dividing the molecular weight of the epoxy compound by the number of moles of epoxy groups included in the epoxy compound.

The bisphenol A-type epoxy resin which can be used in the adhesive according to the embodiment of the present invention is not particularly limited, and any bisphenol A-type epoxy resin commonly used as a main agent of an epoxy-based adhesive can be widely used. Preferred specific examples thereof include bisphenol A diglycidyl ethers (jER825, jER828, and jER834 (all trade names, manufactured by Mitsubishi Chemical Corporation)) and bisphenol A propoxylate diglycidyl ethers (manufactured by Sigma-Aldrich Co. LLC).

The bisphenol F-type epoxy resin which can be used in the adhesive according to the embodiment of the present invention is not particularly limited, and any bisphenol F-type epoxy resin commonly used as a main agent of an epoxy-based adhesive can be widely used. Preferred specific examples thereof include bisphenol F diglycidyl ether (trade name: jER807, manufactured by Mitsubishi Chemical Corporation), bisphenol F diglycidyl ether (trade name: EPICLON 830, manufactured by DIC Corporation) and 4,4'-methylenebis(N,N-diglycidylaniline).

Specific examples of the above-described epoxy resin represented by General Formula (1) include DENACOL EX-810, DENACOL EX-850, DENACOL EX-830, DENACOL EX-211, DENACOL EX-212, DENACOL EX-214L, DENACOL EX-252, DENACOL EX-920, and DENACOL EX-931 (all trade names, manufactured by Nagase ChemteX Corporation); EPOGOSEY HD (trade name, manufactured by Yokkaichi Chemical Co., Ltd.); and 1,6-hexanediol diglycidyl ether and 1,7-octadiene diepoxide (both of which are manufactured by Tokyo Chemical Industry Co., Ltd.).

In the adhesive according to the embodiment of the present invention, a content of the above-described epoxy resin represented by General Formula (1) can be, for example, 1 to 45 parts by mass, preferably 5 to 40 parts by mass, more preferably 10 to 35 parts by mass, and still more preferably 15 to 30 parts by mass with respect to 100 parts by mass of

the total content of the bisphenol A-type epoxy resin and the bisphenol F-type epoxy resin.

A proportion of the total amount of the bisphenol A-type epoxy resin, the bisphenol F-type epoxy resin, and the above-described epoxy resin represented by General Formula (1) with respect to the total amount of the epoxy resin contained in the adhesive according to the embodiment of the present invention can be, for example, 70% by mass or more, preferably 80% by mass or more, more preferably 90% by mass or more, still more preferably 95% by mass or more, and particularly preferably 100% by mass.

The above-described component (a) may include an epoxy resin other than the bisphenol A-type epoxy resin, the bisphenol F-type epoxy resin, and the above-described epoxy resin represented by General Formula (1) (hereinafter referred to as an epoxy resin (x)), as long as the effects of the present invention are not impaired. Specific examples of the epoxy resin (x) include a bisphenol E-type epoxy resin, a hydrogenated bisphenol A-type epoxy resin, and a phenol novolac-type epoxy resin. From the viewpoint of impact resistance of the cured substance according to the embodiment of the present invention, a proportion of the total amount of the epoxy resin (x) contained in the adhesive according to the embodiment of the present invention can be, for example, 10% by mass or less, preferably 5% by mass or less and more preferably 0% by mass.

A content of the epoxy resin contained in the adhesive according to the embodiment of the present invention can be 50% to 90% by mass, more preferably 55% to 80% by mass, still more preferably 55% to 75% by mass, and even more preferably 55% to 70% by mass.

<(b) Imidazole compound having no heteroatom other than ring-constituting nitrogen atom and having melting point of lower than 60°C>

The adhesive according to the embodiment of the present invention contains, as the component (b), an imidazole compound having no heteroatom other than a ring-constituting nitrogen atom and having a melting point of lower than 60°C. Hereinafter, the "imidazole compound having no heteroatom other than a ring-constituting nitrogen atom and having a melting point of lower than 60°C" may be simply referred to as "imidazole compound".

The adhesive according to the embodiment of the present invention may contain only one type of the component (b), or a combination of two or more types of the component (b).

The component (b) has excellent solubility in the epoxy resin and can improve dispersibility of a mixture (preferably, a composition) formed by mixing the respective components contained in the adhesive. In addition, since the component (b) does not have a heteroatom other than a ring-constituting nitrogen atom, in a case of acting on the epoxy resin as a curing component, a stable reaction intermediate with the epoxy resin can be formed. This allows the curing reaction of the epoxy resin to proceed efficiently, thereby improving the impact resistance of the cured substance (cured adhesive substance) according to the embodiment of the present invention. Furthermore, since the melting point of the component (b) is lower than 60°C, the component (b) can be dissolved due to heat generated in a case of mixing the respective components contained in the adhesive according to the embodiment of the present invention, so that it is possible to obtain a composition with excellent uniform dispersibility. The melting point of the imidazole compound of the component (b) is preferably -80°C or higher and lower than 60°C, and more preferably -70°C to 58°C.

Specific examples of the component (b) include 1-methylimidazole (melting point: -60°C), 1-phenylimidazole (melting point: 11°C), 1,2-dimethylimidazole (melting point: 38°C), 1-benzyl-2-phenylimidazole (melting point: 45°C), 4-ethylimidazole (liquid at 20°C), 2-ethyl-4-methylimidazole (melting point: 47°C to 54°C), and 2-butylimidazole (melting point: 52°C).

In the adhesive according to the embodiment of the present invention, since it is possible to impart sufficient viscosity to the mixture obtained by mixing the respective components contained in the adhesive and efficiently proceed the curing reaction of the epoxy resin, the content of the component (b) is 1 to 20 parts by mass, preferably 3 to 18 parts by mass, more preferably 7 to 13 parts by mass, still more preferably 8 to 12 parts by mass, and even more preferably 9 to 11 parts by mass with respect to 100 parts by mass of the total content of the bisphenol A-type epoxy resin and the bisphenol F-type epoxy resin.

<(c) Phenol resin which is liquid at 25°C>

The adhesive according to the embodiment of the present invention contains, as the component (c), a phenol resin which is liquid at 25°C. Hereinafter, the "phenol resin which is liquid at 25°C" may be simply referred to as "phenol resin".

The adhesive according to the embodiment of the present invention may contain only one type of the component (c), or a combination of two or more types of the component (c).

In a case where the component (c) is a liquid at 25°C, sufficient dispersibility can be imparted to the mixture obtained by mixing the respective components contained in the adhesive. In addition, a sufficient viscosity can be imparted to the above-described mixture by a hydrogen bonding between a hydroxyl group of the component (c) and a hydroxyl

group of the component (d), or the like.

A polymerization-average molecular weight of the component (c) is not particularly limited, and can be, for example, 200 to 1,000, preferably 200 to 450.

The component (c) also functions as a curing agent for the epoxy resin.

A weight-average molecular weight of a compound described in the present specification is determined as follows.

The weight-average molecular weight can be measured as a polystyrene-equivalent molecular weight by gel permeation chromatography (GPC).

Specifically, a GPC device HLC-8220 (trade name, manufactured by Tosoh Corporation) is used, tetrahydrofuran is used as an eluent, a column of TSKgel Super AWM-H (trade name, manufactured by Tosoh Corporation) is used, and detection is performed by UV at 23°C and a flow rate of 0.3 to 0.5 mL/min.

Specific examples of the component (c) include MEH-8000H and MEH-8005 (both trade names, manufactured by MEIWAKASEI.,LTD.).

In the adhesive according to the embodiment of the present invention, the content of the component (c) is 1 to 20 parts by mass, preferably 4 to 15 parts by mass, and more preferably 4 to 8 parts by mass with respect to 100 parts by mass of the total content of the bisphenol A-type epoxy resin and the bisphenol F-type epoxy resin.

<(d) Fumed silica having average particle diameter of 5 nm or more and 35 nm or less>

The adhesive according to the embodiment of the present invention contains, as the component (d), fumed silica having an average particle diameter of 5 nm or more and 35 nm or less (preferably, 10 nm or more and 35 nm or less). Hereinafter, the "fumed silica having an average particle diameter of 5 nm or more and 35 nm or less" may be simply referred to as "fumed silica".

The adhesive according to the embodiment of the present invention may contain only one type of the component (d), or a combination of two or more types of the component (d).

The average particle diameter of the component (d) can be determined in the same manner as measurement and calculation methods described in JP2022-47458A.

Specifically, an image captured by a transmission electron microscope (for example, manufactured by Hitachi High-Tech Corporation, HT7800 (trade name)) is analyzed to obtain the average particle diameter. Specifically, 50 images are captured by changing the visual field, 2,500 components (d) are randomly selected, and an average primary particle diameter thereof is obtained by image analysis and calculated as a number average.

In the mixture obtained by mixing the respective components contained in the adhesive, the components (d) can interact with each other to form a higher-order network, thereby imparting sufficient viscosity and thixotropy to the mixture.

Specific examples of the component (d) include AEROSIL OX50, AEROSIL 200, AEROSIL R972, AEROSIL R974, AEROSIL R976, AEROSIL RX50, AEROSIL NAX50, AEROSIL RX200, AEROSIL RX300, AEROSIL R812, AEROSIL RY50, AEROSIL RY200, AEROSIL RY300, AEROSIL R504, AEROSIL R805, and AEROSIL R711 (all trade names, manufactured by Nippon Aerosil Co., Ltd.). These fumed silicas are preferably surface-treated, and preferably have a surface modification group of a trimethylsilyl group or a dimethyl polysiloxane group (for example, a monovalent group in which one hydrogen atom is eliminated from a methyl group of dimethyl polysiloxane), and more preferably have a dimethyl polysiloxane group.

In the adhesive according to the embodiment of the present invention, the content of the component (d) is 1 to 20 parts by mass, preferably 1 to 10 parts by mass, more preferably 3 to 7 parts by mass, and particularly preferably 4 to 6 parts by mass with respect to 100 parts by mass of the total content of the bisphenol A-type epoxy resin and the bisphenol F-type epoxy resin. In addition, the content of the component (d) in the adhesive according to the embodiment of the present invention is preferably 2 to 15 parts by mass, more preferably 3 to 12 parts by mass, and still more preferably 4 to 12 parts by mass.

<(e) Talc>

The adhesive according to the embodiment of the present invention contains talc as the component (e).

The adhesive according to the embodiment of the present invention may contain only one type of the component (e), or a combination of two or more types of the component (e).

In the mixture obtained by mixing the respective components contained in the adhesive, the contact or rubbing between the components (e) suppresses movement of the respective components contained in the composition, and even in a case where the composition is cured at a high temperature of approximtalye 80°C, a decrease in viscosity is suppressed.

A form of the component (e) is not particularly limited and can be, for example, particulate. A compression talc can also be used.

An average particle diameter of the component (e) is not particularly limited, and is, for example, 0.1 to 30 μm, preferably 1 to 20 μm, more preferably 1 to 15 μm, still more preferably 1 to 10 μm, and even more preferably 1 to 6 μm. The average particle diameter of the component (e) is a volume-based median diameter. The median diameter corresponds to 50% cumulative distribution in a case where the particle diameter distribution is represented as a cumulative distribution. The average particle diameter of the component (d) can be determined in the same manner as the measurement method described in JP2007-197716A.

Specifically, 0.1 g of talc micropowder is taken and put into a 100 mL glass beaker. 50 mL of a 1% by mass aqueous solution of an anionic surfactant (sodium dodecylbenzenesulfonate) is added to the glass beaker. The glass beaker is placed in an ultrasonic cleaner and continuously dispersed for 1 minute with ultrasonic waves having an oscillation frequency of 45 kHz and a rated output of 100 W to prepare a measurement sample. The measurement is performed using a laser diffraction-type particle size distribution analyzer (product name "SALD-2000J" manufactured by Shimadzu Corporation).

Specific examples of the component (e) include MICRO ACE SG95, MICRO ACE P-3, MICRO ACE K-1, MICRO ACE MS-P, SG-200, SG-2000, NANO ACE D-600, and NANO ACE D-1000 (all trade names, manufactured by Nippon Talc Co.,Ltd.); and Crown Talc HS, Crown Talc 250M, and Hifiller #17 (all trade names, manufactured by Matsumura sangyo Co., Ltd.).

From the viewpoint of effectively imparting sufficient viscosity to the mixture obtained by mixing the respective components contained in the adhesive and effectively suppressing color unevenness of the cured adhesive substance, the content of component (d) in the adhesive according to the embodiment of the present invention is 5 to 30 parts by mass, preferably 10 to 25 parts by mass, more preferably 15 to 25 parts by mass, and still more preferably 18 to 23 parts by mass with respect to 100 parts by mass of the total content of the bisphenol A-type epoxy resin and the bisphenol F-type epoxy resin.

<(f) Silane coupling agent>

The adhesive according to the embodiment of the present invention preferably contains a silane coupling agent as a component (f). The component (f) can be incorporated into the cured substance by reacting with the epoxy resin during the curing of the adhesive according to the embodiment of the present invention, and can contribute to the improvement and maintenance of the adhesive force to the constituting member of the medical device.

The adhesive according to the embodiment of the present invention may contain only one type of the component (f), or a combination of two or more types of the component (f).

As the component (f), for example, KBM series manufactured by Shin-Etsu Silicone Co., Ltd. can be used, and specific examples thereof include KBE-1003, KBM-1403, KBE-503, KBM-5103, KBM-403, KBM-903, KBM-9659, and KBM-802.

In the adhesive according to the embodiment of the present invention, a content of the component (f) can be, for example, 1 to 10 parts by mass, preferably 2 to 6 parts by mass with respect to 100 parts by mass of the total content of the bisphenol A-type epoxy resin and the bisphenol F-type epoxy resin.

<(g) Other components>

The adhesive according to the embodiment of the present invention may contain, as a component (g), a component other than the above-described components (a) to (f), as long as the effects of the present invention are not impaired. Specific examples of the component (g) include a colorant (for example, carbon black), a solvent, a plasticizer, an adhesion strength enhancer, a dispersion stabilizer, an ion supplementing agent (for example, a cation exchanger, an anion exchanger, or a zwitterion exchanger), and a viscosity adjuster.

The adhesive according to the embodiment of the present invention may contain only one type of the component (g), or a combination of two or more types of the component (g). Among these, the adhesive according to the embodiment of the present invention preferably contains a colorant.

In the adhesive according to the embodiment of the present invention, a content of the component (g) can be, for example, 1 to 10 parts by mass, preferably 3 to 7 parts by mass with respect to 100 parts by mass of the total content of the bisphenol A-type epoxy resin and the bisphenol F-type epoxy resin.

From the viewpoint of effective interaction between the respective components, the total content of the components (b) and (c) in the adhesive according to the embodiment of the present invention is preferably 5 parts by mass or more and less than 30 parts by mass, and more preferably 10 parts by mass or more and 24 parts by mass or less with respect to 100 parts by mass of the total content of the bisphenol A-type epoxy resin and the bisphenol F-type epoxy resin.

From the viewpoint of effective interaction between the respective components, a value of a ratio of the content of component (c) to the content of component (b) (Content of component (c)/Content of component (b) (mass ratio)) in the

adhesive according to the embodiment of the present invention is preferably 0.1 to 2.0 and more preferably 0.3 to 1.0.

[Cured substance]

The cured substance according to the embodiment of the present invention is a cured substance obtained by curing the adhesive according to the embodiment of the present invention. That is, the cured substance according to the embodiment of the present invention is used as a member constituting an adhesive portion of a medical device or a constituting member of a medical device. A curing temperature of the adhesive according to the embodiment of the present invention is not particularly limited, and can be appropriately set according to the component (b) contained in the adhesive according to the embodiment of the present invention. The mixing of the respective components can be performed by a conventional method. It is preferable that the mixing is performed while removing air bubbles, and thus the mixing is usually performed under reduced pressure.

Specifically, the adhesive according to the embodiment of the present invention undergoes an efficient curing reaction over a wide temperature range from a low temperature to a high temperature, so that the cured substance according to the embodiment of the present invention can be obtained. The above-described curing temperature can be, for example, 150°C or lower, 120°C or lower, 100°C or lower, or 85°C or lower. In addition, in order to sufficiently perform the curing reaction, the curing temperature can be 15°C or higher, 50°C or higher, or 80°C or higher. A curing reaction time can be appropriately set according to the purpose. The curing reaction is usually performed for 1.5 to 200 hours to obtain the cured substance.

[Medical device member]

Fig. 1 is a cross-sectional view schematically showing an embodiment of the medical device member (sheet-shaped medical device member) according to the embodiment of the present invention.

Hereinafter, a medical device member 10 according to the embodiment of the present invention (hereinafter, the "medical device member" is also simply referred to as "member") includes a substrate 1 and a cured substance 2 (cured substance (sheet) according to the embodiment of the present invention) on the substrate.

The above-described reference numerals correspond to reference numerals in Fig. 1.

From the viewpoint of making mechanical properties substantially uniform over the entire member, in the medical device member 10 according to the embodiment of the present invention, a value of a ratio of a magnesium element amount on a surface of the above-described sheet in contact with the substrate to a magnesium element amount on a surface of the above-described sheet on a side opposite to the above-described substrate is preferably 1.0 to 1.3, and more preferably 1.0 to 1.2.

The "magnesium element amount" is a value determined by a method described in the section of Examples later.

<Substrate>

The substrate included in the medical device member according to the embodiment of the present invention is not particularly limited, and a s used as a conventional constituting member of a medical device can be widely adopted.

Specifically, the substrate can include, for example, a metal (iron and non-ferrous metal), an inorganic material other than metal, and an organic material.

The above-described iron also includes alloys of iron and nonferrous metals. Examples of such an alloy include stainless steel.

Examples of the above-described non-ferrous metal include aluminum, titanium, magnesium, nickel, copper, lead, zinc, tin, chromium, tungsten, cobalt, vanadium, and gold, and alloys of at least two of these metals, and aluminum, titanium, magnesium, nickel, copper, lead, zinc, tin, chromium, tungsten, cobalt, or alloys of at least two of these metals are preferable.

Examples of the inorganic material other than the metals described above include glass and glass ceramics.

Examples of the above-described glass include sodium soda glass, Pyrex (registered trademark) glass, quartz glass, and non-alkali glass.

Examples of the above-described ceramics include alumina, zirconia, silicon carbide, and silicon nitride.

Examples of the above-described organic material include a resin (a thermoplastic resin and a thermosetting resin).

Examples of the above-described thermoplastic resin include a thermoplastic polyimide resin, a thermoplastic polyamide resin, a polyetherimide resin, a polyphenylene ether resin, a polycarbonate resin, a polyethylene terephthalate resin, a polyethylene naphthalate resin, a polyphenylene sulfide resin, a polyether ether ketone resin, a polyethersulfone resin, an acrylic resin, polyolefin resins such as a polyethylene resin, a polypropylene resin, and a polymethylpentene resin, and thermoplastic polycycloolefins such as thermoplastic polynorbornene.

Examples of the above-described thermosetting resin include a thermosetting polyimide resin, a thermosetting polyamide resin, a polyamidoimide resin, an epoxy resin, a phenol resin, a polystyrene resin, an acrylonitrile-butadiene-styrene copolymer resin (ABS resin), styrene resins such as an acrylonitrile-styrene copolymer resin, and thermosetting polycycloolefins such as thermosetting polynorbornene.

Among these, as the substrate included in the medical device member according to the embodiment of the present invention, a resin substrate, a glass substrate, or a stainless steel substrate is preferable.

Physical properties of the substrate, such as flexibility and rigidity, can be appropriately determined according to the medical device to which the member is applied. The same applies to a thickness of the substrate. The thickness of the substrate may be, for example, 0.1 to 50 mm, or 0.5 to 10 mm.

In addition, a shape of the substrate is not particularly limited, and the substrate may have an uneven shape or may have a concave portion. Since the adhesive according to the embodiment of the present invention has excellent shape retention, a thickly coated shape can be maintained while being cured. Therefore, for example, the sealing can be performed with high accuracy, regardless of the shape of the concave portion of the substrate. In addition, a desired structure (such as a desired protrusion shape) can also be formed by curing the adhesive. That is, the adhesive according to the embodiment of the present invention is also suitable as a resin material or a resin composition for obtaining a resin structure (resin molded article).

A content of at least one of the metal, the inorganic material other than the metal, or the organic material, contained in the substrate, is not particularly limited, and may be, for example, 80% by mass or more, preferably 90% by mass or more, and may be 100% by mass.

[Medical device]

Examples of a medical device in which a constituting member is fixed by the cured substance according to the embodiment of the present invention, and a medical device to which the member according to the embodiment of the present invention can be applied, that is, the medical device according to the embodiment of the present invention include a catheter, an applicator, an X-ray imaging device, an electric surgical instrument, a treatment actuator, an ultrasound diagnostic apparatus, and an endoscope.

[Endoscope]

In one aspect of the endoscope of the present invention, the medical device member according to the embodiment of the present invention is provided as a constituting member.

In another aspect of the endoscope of the present invention, the constituting member is fixed by the cured substance according to the embodiment of the present invention. The phrase "the constituting member is fixed by the cured substance according to the embodiment of the present invention" means that at least a part of members constituting the endoscope is fixed to the support member through the cured substance according to the embodiment of the present invention.

[Manufacturing method of medical device]

The medical device according to the embodiment of the present invention can be manufactured by a common method, except that the adhesive according to the embodiment of the present invention is used, and for example, the medical device can be manufactured with reference to WO2020/175272A, JP2009-194934A, JP2014-511191A, and JP2001-128929A.

Examples

The present invention will be described in more detail with reference to Examples. The present invention is not limited to Examples, except as specified in the present invention.

<Preparation of adhesive>

(Preparation of adhesive 1 (adhesive of Example 1 in Table 1 described later))

100 parts by mass of bisphenol A diglycidyl ether (product name: jER828, manufactured by Mitsubishi Chemical Corporation, epoxy equivalent: 189) (component a-1 in Table 1 described later), 10 parts by mass of 2-ethyl-4-methylimidazole (component b-1 in Table 1 described later), 5 parts by mass of a phenol resin (trade name: MEH-8000H, manufactured by Showa Kasei Kogyo Co., Ltd.) (component c-1 in Table 1 described later), 5 parts by mass of fumed silica

(product name: AEROSIL RY-200, manufactured by Nippon Aerosil Co., Ltd., average particle diameter: 12 nm) (component d-3 in Table 1 described later), 20 parts by mass of talc (trade name: SG-95, average particle diameter: 2.5 μm, manufactured by Nippon Talc Co.,Ltd.) (component e-1 in Table 1 described later), and 5 parts by mass of carbon black (component Carbon in Table 1 described later) were stirred with "AWATORIRENTARO ARV-310 (trade name, manufactured by THINKY)" with 2,000 rpm in a reduced pressure state of 25°C and 1.0 Pa for 5 minutes to be defoamed, thereby obtaining an adhesive 1.

The "Table 1" means the following tables 1-1 to 1-6.

(Preparation of adhesives 2 to 38 and c1 to c27 (adhesives of Examples 2 to 38 and Comparative Examples 1 to 27 in Table 1 described later))

Adhesives 2 to 38 and c1 to c27 were prepared in the same manner as the adhesive 1, except that the composition of the adhesive 1 was changed to compositions of the adhesives 2 to 38 and c1 to c27 as shown in Table 1 described later.

[Measurement and test]

The following measurements and tests (Test Examples 1 to 4) were performed on the prepared adhesives 2 to 38 and c1 to c27. The results are summarized in Table 1.

[Measurement of Mg element amount and calculation of value of ratio of Mg element amounts]

The adhesive was poured into a Teflon (registered trademark)-made mold frame (thickness: 1 mm, vertical: 2 cm × horizontal: 2 cm) placed on a Teflon sheet (thickness: 1 mm, manufactured by MISUMI Corporation) (substrate), and cured at 80°C for 15 hours. The Teflon-made mold frame was removed, and a sheet-shaped cured substance was peeled off from the Teflon sheet.

An Mg element amount on a surface of the sheet-shaped cured substance on a side opposite to the substrate was quantified by scanning electron microscope-energy dispersive X-ray analysis (SEM-EDX). As an SEM-EDX device, a tabletop microscope Miniscope TM4000 Plus II (trade name, manufactured by Hitachi High-Tech Corporation) was used.

The surface analysis was carried out for each 4 $mm^2$ region at an acceleration voltage of 15 kV to obtain a value (Mg value (T)) by summing signal intensities of the Mg element over the entire surface.

The surface of the sheet-shaped cured substance on the substrate side was subjected to surface analysis in the same manner as described above, and a value (Mg value (B)) obtained by summing signal intensities of the Mg element over the entire surface was obtained.

From the following expression, the value of the ratio of the Mg element amounts in the sheet-shaped cured substance was calculated.

$$\text{Value of the ratio of Mg element amounts} = \text{Mg value (B)/Mg value (T)}$$

Even in a case where a material of the substrate was changed, the value of the ratio of the Mg element amounts could be obtained in the same manner as described above.

[Test Example 1: antibacterial performance]

The adhesive was applied onto a urethane sheet (manufactured by Standard Test Piece Co., Ltd.) (substrate) having a thickness of 3 mm, a width of 5 cm, and a length of 10 cm with a thickness of 0.5 mm, a width of 3 cm, and a length of 5 cm, and cured at 80°C for 12 hours to obtain a medical device member. After the medical device member was allowed to stand until the temperature of the medical device member reached room temperature, the medical device member was immersed in a 1% by mass aqueous solution of a detergent (CHARMY GREEN (trade name), manufactured by Lion Corporation) at 25°C for 30 minutes and washed with tap water to remove the attached detergent.

After wiping off water droplets from the medical device member, the medical device member was placed into a high-temperature and high-humidity tank at 40°C and 50 %RH (relative humidity). The time after being placed in the high-temperature and high-humidity tank until mold started to grow was observed with the naked eye, and evaluated according to the following evaluation standard.

-Evaluation standard-

S: no mold was grown even after 1,000 hours.

A: mold started to grow at a peripheral edge portion of the cured adhesive substance (boundary between the urethane sheet and the cured adhesive substance) in 800 hours or more and less than 1,000 hours

B: mold started to grow at the peripheral edge portion of the cured adhesive substance in 600 hours or more and less than 800 hours

C: mold started to grow at the peripheral edge portion of the cured adhesive substance in 400 hours or more and less than 600 hours

D: mold started to grow at the peripheral edge portion of the cured adhesive substance in less than 400 hours

[Test Example 2: color unevenness]

A circular pipe (substrate) made of stainless steel (SUS) 304 with an inner diameter of 5 mm, an outer diameter of 9 mm, and a length of 50 mm was covered with a polyurethane tube (substrate) having an inner diameter of 9 mm and an outer diameter of 10 mm, and the adhesive was applied to an outer periphery of the tube in a width of 5 mm and a thickness of 1 mm.

The pipe to which the adhesive had been applied was heated at 80°C for 12 hours with a length direction kept horizontal to cure the adhesive, thereby obtaining a medical device member. The cured adhesive substance constituting the medical device member was visually observed with a loupe, and evaluated according to the following evaluation standard.

-Evaluation standard-

A: there was no color unevenness
B: there was a slightly transparent portion at an edge of the cured adhesive substance (boundary between the cured adhesive substance and the polyurethane tube)
C: sedimentation of an obvious colorant (carbon black) was observed at the edge of the cured adhesive substance
D: sedimentation of an obvious colorant (carbon black) was observed in the vicinity of the center of the cured adhesive substance in the width direction

[Test Example 3: impact resistance]

A circular pipe (substrate) made of SUS 304 with an inner diameter of 15 mm, an outer diameter of 18 mm, and a length of 50 mm was covered with a polyurethane tube (substrate) having an inner diameter of 18 mm and an outer diameter of 20 mm, and the adhesive was applied to an outer periphery of the tube in a width of 15 mm and a thickness of 1 mm.

The pipe to which the adhesive had been applied was heated at 80°C for 12 hours with a length direction kept horizontal to cure the adhesive, thereby obtaining a medical device member.

The medical device member was set in a drop ball tester (trade name: No. 183 drop ball impact tester) manufactured by Yasuda Seiki Co., Ltd. The member for a medical device was subjected to a vertical drop test in which a SUS ball with a diameter of 20 mm (weight: 0.033 kg, manufactured by E-Metals Co., Ltd.) was dropped from a height of 30 cm to collide with the cured adhesive substance of the member for a medical device. This test was repeated, and the number of times the first damage (crack or breakage) occurred in the cured substance of the medical device member was evaluated according to the following evaluation standard.

-Evaluation standard-

A: more than 20 times
B: 10 to 20 times
C: less than 10 times

[Test Example 4: adhesiveness]

The adhesive was applied onto an outer periphery of a circular pipe (substrate) made of SUS303 with an inner diameter of 5 mm, an outer diameter of 9 mm, and a length of 50 mm, in a width of 5 mm and a thickness of 1 mm. The pipe to which the adhesive had been applied was heated at 80°C for 12 hours with a length direction kept horizontal to cure the

adhesive, thereby obtaining a medical device member. The medical device member was placed in an oven at 70°C for 2,500 hours, and allowed to stand until the temperature was returned to room temperature. A boundary between the substrate and the cured adhesive substance of the medical device member after standing was observed with a loupe, and evaluated according to the following evaluation standard.

-Evaluation standard-

A: the cured adhesive substance and the substrate was closely adhered to each other
B: the cured adhesive substance was slightly floating
C: peeling of the cured adhesive substance from the substrate occurred

[Table 1-1]

| Component | Melting point (°C) | E1 | E2 | E3 | E4 | E5 | E6 | E7 | E8 | E9 | E10 | E11 |
|---|---|---|---|---|---|---|---|---|---|---|---|---|
| a-1 | | 100 | -- | 100 | 100 | 100 | 100 | 100 | 100 | 100 | 100 | 100 |
| a-2 | | -- | 100 | -- | -- | -- | -- | -- | -- | -- | -- | -- |
| a-3 | | -- | -- | -- | -- | -- | -- | -- | -- | -- | -- | -- |
| b-1 | 47 to 54 | 10 | 10 | -- | -- | -- | -- | -- | 10 | 10 | 10 | 10 |
| b-2 | < 20 | -- | -- | 10 | -- | -- | -- | -- | -- | -- | -- | -- |
| b-3 | -60 | -- | -- | -- | 10 | -- | -- | -- | -- | -- | -- | -- |
| b-4 | 13 | -- | -- | -- | -- | 10 | -- | -- | -- | -- | -- | -- |
| b-5 | 38 | -- | -- | -- | -- | -- | 10 | -- | -- | -- | -- | -- |
| b-6 | 52 | -- | -- | -- | -- | -- | -- | 10 | -- | -- | -- | -- |
| b-7 | 154 | -- | -- | -- | -- | -- | -- | -- | -- | -- | -- | -- |
| b-8 | 11 | -- | -- | -- | -- | -- | -- | -- | -- | -- | -- | -- |
| b-9 | 142 | -- | -- | -- | -- | -- | -- | -- | -- | -- | -- | -- |
| b-10 | < 20 | -- | -- | -- | -- | -- | -- | -- | -- | -- | -- | -- |
| b-11 | 117 | -- | -- | -- | -- | -- | -- | -- | -- | -- | -- | -- |
| b-12 | 200 < | -- | -- | -- | -- | -- | -- | -- | -- | -- | -- | -- |
| b-13 | | -- | -- | -- | -- | -- | -- | -- | -- | -- | -- | -- |
| c-1 | | 5 | 5 | 5 | 5 | 5 | 5 | 5 | -- | 5 | 5 | 5 |
| c-2 | | -- | -- | -- | -- | -- | -- | -- | 5 | -- | -- | -- |
| c-3 | | -- | -- | -- | -- | -- | -- | -- | -- | -- | -- | -- |
| d-1 | | -- | -- | -- | -- | -- | -- | -- | -- | 5 | -- | -- |
| d-2 | | -- | -- | -- | -- | -- | -- | -- | -- | -- | 5 | -- |
| d-3 | | 5 | 5 | 5 | 5 | 5 | 5 | 5 | 5 | -- | -- | 5 |
| d-4 | | -- | -- | -- | -- | -- | -- | -- | -- | -- | -- | -- |
| d-5 | | -- | -- | -- | -- | -- | -- | -- | -- | -- | -- | -- |
| e-1 | | 20 | 20 | 20 | 20 | 20 | 20 | 20 | 20 | 20 | 20 | 20 |
| e-2 | | -- | -- | -- | -- | -- | -- | -- | -- | -- | -- | -- |
| e-3 | | -- | -- | -- | -- | -- | -- | -- | -- | -- | -- | -- |
| e-4 | | -- | -- | -- | -- | -- | -- | -- | -- | -- | -- | -- |

(continued)

| Component | Melting point (°C) | E1 | E2 | E3 | E4 | E5 | E6 | E7 | E8 | E9 | E10 | E11 |
|---|---|---|---|---|---|---|---|---|---|---|---|---|
| e-5 | | -- | -- | -- | -- | -- | -- | -- | -- | -- | -- | -- |
| e-6 | | -- | -- | -- | -- | -- | -- | -- | -- | -- | -- | -- |
| Carbon | | 5 | 5 | 5 | 5 | 5 | 5 | 5 | 5 | 5 | 5 | 5 |
| f-1 | | -- | -- | -- | -- | -- | -- | -- | -- | -- | -- | -- |
| x-1 | | -- | -- | -- | -- | -- | -- | -- | -- | -- | -- | -- |
| x-2 | | -- | -- | -- | -- | -- | -- | -- | -- | -- | -- | -- |
| Mg element amount | | 1.14 | 1.14 | 1.14 | 1.14 | 1.20 | 1.14 | 1.20 | 1.25 | 1.20 | 1.20 | 1.14 |
| Antibacterial performance | | S | S | S | S | A | S | A | B | A | A | S |
| Color unevenness | | A | A | A | A | A | A | A | A | A | B | A |
| Impact resistance | | B | B | B | B | B | B | B | B | B | B | B |
| Adhesiveness | | B | B | B | B | B | B | B | B | B | B | B |

[Table 1-2]

| Component | Melting point (°C) | E12 | E13 | E14 | E15 | E16 | E17 | E18 | E19 | E20 | E21 | E22 |
|---|---|---|---|---|---|---|---|---|---|---|---|---|
| a-1 | | 100 | 100 | 100 | 100 | 100 | 100 | 100 | 100 | 100 | 100 | 100 |
| a-2 | | -- | -- | -- | -- | -- | -- | -- | -- | -- | -- | -- |
| a-3 | | -- | -- | -- | -- | -- | -- | -- | -- | -- | -- | -- |
| b-1 | 47 to 54 | 10 | 10 | 10 | 1 | 5 | 8 | 12 | 15 | 20 | 10 | 10 |
| b-2 | < 20 | -- | -- | -- | -- | -- | -- | -- | -- | -- | -- | -- |
| b-3 | -60 | -- | -- | -- | -- | -- | -- | -- | -- | -- | -- | -- |
| b-4 | 13 | -- | -- | -- | -- | -- | -- | -- | -- | -- | -- | -- |
| b-5 | 38 | -- | -- | -- | -- | -- | -- | -- | -- | -- | -- | -- |
| b-6 | 52 | -- | -- | -- | -- | -- | -- | -- | -- | -- | -- | -- |
| b-7 | 154 | -- | -- | -- | -- | -- | -- | -- | -- | -- | -- | -- |
| b-8 | 11 | -- | -- | -- | -- | -- | -- | -- | -- | -- | -- | -- |
| b-9 | 142 | -- | -- | -- | -- | -- | -- | -- | -- | -- | -- | -- |
| b-10 | < 20 | -- | -- | -- | -- | -- | -- | -- | -- | -- | -- | -- |
| b-11 | 117 | -- | -- | -- | -- | -- | -- | -- | -- | -- | -- | -- |
| b-12 | 200 < | -- | -- | -- | -- | -- | -- | -- | -- | -- | -- | -- |
| b-13 | | -- | -- | -- | -- | -- | -- | -- | -- | -- | -- | -- |
| c-1 | | 5 | 5 | 5 | 5 | 5 | 5 | 5 | 5 | 5 | 1 | 3 |
| c-2 | | -- | -- | -- | -- | -- | -- | -- | -- | -- | -- | -- |
| c-3 | | -- | -- | -- | -- | -- | -- | -- | -- | -- | -- | -- |
| d-1 | | -- | -- | -- | -- | -- | -- | -- | -- | -- | -- | -- |
| d-2 | | -- | -- | -- | -- | -- | -- | -- | -- | -- | -- | -- |

(continued)

| Component | Melting point (°C) | E12 | E13 | E14 | E15 | E16 | E17 | E18 | E19 | E20 | E21 | E22 |
|---|---|---|---|---|---|---|---|---|---|---|---|---|
| d-3 | | 5 | 5 | 5 | 5 | 5 | 5 | 5 | 5 | 5 | 5 | 5 |
| d-4 | | -- | -- | -- | -- | -- | -- | -- | -- | -- | -- | -- |
| d-5 | | -- | -- | -- | -- | -- | -- | -- | -- | -- | -- | -- |
| e-1 | | -- | -- | -- | 20 | 20 | 20 | 20 | 20 | 20 | 20 | 20 |
| e-2 | | 20 | -- | -- | -- | -- | -- | -- | -- | -- | -- | -- |
| e-3 | | -- | 20 | -- | -- | -- | -- | -- | -- | -- | -- | -- |
| e-4 | | -- | -- | 20 | -- | -- | -- | -- | -- | -- | -- | -- |
| e-5 | | -- | -- | -- | -- | -- | -- | -- | -- | -- | -- | -- |
| e-6 | | -- | -- | -- | -- | -- | -- | -- | -- | -- | -- | -- |
| Carbon | | 5 | 5 | 5 | 5 | 5 | 5 | 5 | 5 | 5 | 5 | 5 |
| f-1 | | -- | -- | -- | -- | -- | -- | -- | -- | -- | -- | -- |
| x-1 | | -- | -- | -- | -- | -- | -- | -- | -- | -- | -- | -- |
| x-2 | | -- | -- | -- | -- | -- | -- | -- | -- | -- | -- | -- |
| Mg element amount | | 1.14 | 1.20 | 1.20 | 1.29 | 1.25 | 1.20 | 1.20 | 1.27 | 1.29 | 1.29 | 1.27 |
| Antibacterial performance | | S | A | A | C | B | A | A | B | C | C | B |
| Color unevenness | | A | A | A | A | A | A | A | B | C | C | B |
| Impact resistance | | B | B | B | B | B | B | B | B | B | B | B |
| Adhesiveness | | B | B | B | B | B | B | B | B | B | B | B |

[Table 1-3]

| Component | Melting point (°C) | E23 | E24 | E25 | E26 | E27 | E28 | E29 | E30 | E31 | E32 | E33 |
|---|---|---|---|---|---|---|---|---|---|---|---|---|
| a-1 | | 100 | 100 | 100 | 100 | 100 | 100 | 100 | 100 | 100 | 100 | 100 |
| a-2 | | -- | -- | -- | -- | -- | -- | -- | -- | -- | -- | -- |
| a-3 | | -- | -- | -- | -- | -- | -- | -- | -- | -- | -- | 5 |
| b-1 | 47 to 54 | 10 | 10 | 10 | 10 | 10 | 10 | 10 | 10 | 10 | 10 | 10 |
| b-2 | < 20 | -- | -- | -- | -- | -- | -- | -- | -- | -- | -- | -- |
| b-3 | -60 | -- | -- | -- | -- | -- | -- | -- | -- | -- | -- | -- |
| b-4 | 13 | -- | -- | -- | -- | -- | -- | -- | -- | -- | -- | -- |
| b-5 | 38 | -- | -- | -- | -- | -- | -- | -- | -- | -- | -- | -- |
| b-6 | 52 | -- | -- | -- | -- | -- | -- | -- | -- | -- | -- | -- |
| b-7 | 154 | -- | -- | -- | -- | -- | -- | -- | -- | -- | -- | -- |
| b-8 | 11 | -- | -- | -- | -- | -- | -- | -- | -- | -- | -- | -- |
| b-9 | 142 | -- | -- | -- | -- | -- | -- | -- | -- | -- | -- | -- |
| b-10 | < 20 | -- | -- | -- | -- | -- | -- | -- | -- | -- | -- | -- |
| b-11 | 117 | -- | -- | -- | -- | -- | -- | -- | -- | -- | -- | -- |

(continued)

| Component | Melting point (°C) | E23 | E24 | E25 | E26 | E27 | E28 | E29 | E30 | E31 | E32 | E33 |
|---|---|---|---|---|---|---|---|---|---|---|---|---|
| b-12 | 200 < | -- | -- | -- | -- | -- | -- | -- | -- | -- | -- | -- |
| b-13 | | -- | -- | -- | -- | -- | -- | -- | -- | -- | -- | -- |
| c-1 | | 10 | 20 | 5 | 5 | 5 | 5 | 5 | 5 | 5 | 5 | 10 |
| c-2 | | -- | -- | -- | -- | -- | -- | -- | -- | -- | -- | -- |
| c-3 | | -- | -- | -- | -- | -- | -- | -- | -- | -- | -- | -- |
| d-1 | | -- | -- | -- | -- | -- | -- | -- | -- | -- | -- | -- |
| d-2 | | -- | -- | -- | -- | -- | -- | -- | -- | -- | -- | -- |
| d-3 | | 5 | 5 | 1 | 3 | 10 | 20 | 5 | 5 | 5 | 5 | 5 |
| d-4 | | -- | -- | -- | -- | -- | -- | -- | -- | -- | -- | -- |
| d-5 | | -- | -- | -- | -- | -- | -- | -- | -- | -- | -- | -- |
| e-1 | | 20 | 20 | 20 | 20 | 20 | 20 | 5 | 10 | 15 | 25 | 20 |
| e-2 | | -- | -- | -- | -- | -- | -- | -- | -- | -- | -- | -- |
| e-3 | | -- | -- | -- | -- | -- | -- | -- | -- | -- | -- | -- |
| e-4 | | -- | -- | -- | -- | -- | -- | -- | -- | -- | -- | -- |
| e-5 | | -- | -- | -- | -- | -- | -- | -- | -- | -- | -- | -- |
| e-6 | | -- | -- | -- | -- | -- | -- | -- | -- | -- | -- | -- |
| Carbon | | 5 | 5 | 5 | 5 | 5 | 5 | 5 | 5 | 5 | 5 | 5 |
| f-1 | | -- | -- | -- | -- | -- | -- | -- | -- | -- | -- | -- |
| x-1 | | -- | -- | -- | -- | -- | -- | -- | -- | -- | -- | -- |
| x-2 | | -- | -- | -- | -- | -- | -- | -- | -- | -- | -- | -- |
| Mg element amount | | 1.20 | 1.28 | 1.29 | 1.27 | 1.25 | 1.28 | 1.29 | 1.27 | 1.20 | 1.25 | 1.20 |
| Antibacterial performance | | A | C | C | B | B | C | C | B | A | B | A |
| Color unevenness | | A | C | B | B | A | C | B | B | A | A | A |
| Impact resistance | | B | B | B | B | B | B | B | B | B | B | A |
| Adhesiveness | | B | B | B | B | B | B | B | B | B | B | B |

[Table 1-4]

| Component | Melting point (°C) | E34 | E35 | E36 | E37 | E38 | C1 | C2 | C3 | C4 | C5 | C6 |
|---|---|---|---|---|---|---|---|---|---|---|---|---|
| a-1 | | 100 | 100 | 100 | 100 | 100 | 100 | 100 | 100 | 100 | 100 | 100 |
| a-2 | | -- | -- | -- | -- | -- | -- | -- | -- | -- | -- | -- |
| a-3 | | 20 | 30 | 40 | 5 | 20 | -- | -- | -- | -- | -- | -- |
| b-1 | 47 to 54 | 10 | 10 | 10 | 10 | 10 | 0.5 | 25 | -- | -- | -- | -- |
| b-2 | < 20 | -- | -- | -- | -- | -- | -- | -- | -- | -- | -- | -- |
| b-3 | -60 | -- | -- | -- | -- | -- | -- | -- | -- | -- | -- | -- |
| b-4 | 13 | -- | -- | -- | -- | -- | -- | -- | -- | -- | -- | -- |

(continued)

| Component | Melting point (°C) | E34 | E35 | E36 | E37 | E38 | C1 | C2 | C3 | C4 | C5 | C6 |
|---|---|---|---|---|---|---|---|---|---|---|---|---|
| b-5 | 38 | -- | -- | -- | -- | -- | -- | -- | -- | -- | -- | -- |
| b-6 | 52 | -- | -- | -- | -- | -- | -- | -- | -- | -- | -- | -- |
| b-7 | 154 | -- | -- | -- | -- | -- | -- | -- | 10 | -- | -- | -- |
| b-8 | 11 | -- | -- | -- | -- | -- | -- | -- | -- | 10 | -- | -- |
| b-9 | 142 | -- | -- | -- | -- | -- | -- | -- | -- | -- | 10 | -- |
| b-10 | < 20 | -- | -- | -- | -- | -- | -- | -- | -- | -- | -- | 10 |
| b-11 | 117 | -- | -- | -- | -- | -- | -- | -- | -- | -- | -- | -- |
| b-12 | 200 < | -- | -- | -- | -- | -- | -- | -- | -- | -- | -- | -- |
| b-13 | | -- | -- | -- | -- | -- | -- | -- | -- | -- | -- | -- |
| c-1 | | 10 | 10 | 10 | 10 | 10 | 5 | 5 | 5 | 5 | 5 | 5 |
| c-2 | | -- | -- | -- | -- | -- | -- | -- | -- | -- | -- | -- |
| c-3 | | -- | -- | -- | -- | -- | -- | -- | -- | -- | -- | -- |
| d-1 | | -- | -- | -- | -- | -- | -- | -- | -- | -- | -- | -- |
| d-2 | | -- | -- | -- | -- | -- | -- | -- | -- | -- | -- | -- |
| d-3 | | 5 | 5 | 5 | 5 | 5 | 5 | 5 | 5 | 5 | 5 | 5 |
| d-4 | | -- | -- | -- | -- | -- | -- | -- | -- | -- | -- | -- |
| d-5 | | -- | -- | -- | -- | -- | -- | -- | -- | -- | -- | -- |
| e-1 | | 20 | 20 | 20 | 20 | 20 | 20 | 20 | 20 | 20 | 20 | 20 |
| e-2 | | -- | -- | -- | -- | -- | -- | -- | -- | -- | -- | -- |
| e-3 | | -- | -- | -- | -- | -- | -- | -- | -- | -- | -- | -- |
| e-4 | | -- | -- | -- | -- | -- | -- | -- | -- | -- | -- | -- |
| e-5 | | -- | -- | -- | -- | -- | -- | -- | -- | -- | -- | -- |
| e-6 | | -- | -- | -- | -- | -- | -- | -- | -- | -- | -- | -- |
| Carbon | | 5 | 5 | 5 | 5 | 5 | 5 | 5 | 5 | 5 | 5 | 5 |
| f-1 | | -- | -- | -- | 5 | 3 | -- | -- | -- | -- | -- | -- |
| x-1 | | -- | -- | -- | -- | -- | -- | -- | -- | -- | -- | -- |
| x-2 | | -- | -- | -- | -- | -- | -- | -- | -- | -- | -- | -- |
| Mg element amount | | 1.14 | 1.14 | 1.20 | 1.10 | 1.10 | 1.42 | 1.38 | 1.40 | 1.40 | 1.40 | 1.40 |
| Antibacterial performance | | S | S | A | S | S | D | D | D | D | D | D |
| Color unevenness | | A | A | A | A | A | C | C | C | C | C | C |
| Impact resistance | | A | A | A | A | A | C | C | C | C | C | C |
| Adhesiveness | | B | B | B | A | A | C | C | C | C | C | C |

[Table 1-5]

| Component | Melting point (°C) | C7 | C8 | C9 | C10 | C11 | C12 | C13 | C14 | C15 | C16 | C17 |
|---|---|---|---|---|---|---|---|---|---|---|---|---|
| a-1 | | 100 | 100 | 100 | 100 | 100 | 100 | 100 | 100 | 100 | 100 | 100 |
| a-2 | | -- | -- | -- | -- | -- | -- | -- | -- | -- | -- | -- |
| a-3 | | -- | -- | -- | -- | -- | -- | -- | -- | -- | -- | -- |
| b-1 | 47 to 54 | -- | -- | 10 | 10 | 10 | 10 | 10 | 10 | 10 | 10 | 10 |
| b-2 | < 20 | -- | -- | -- | -- | -- | -- | -- | -- | -- | -- | -- |
| b-3 | -60 | -- | -- | -- | -- | -- | -- | -- | -- | -- | -- | -- |
| b-4 | 13 | -- | -- | -- | -- | -- | -- | -- | -- | -- | -- | -- |
| b-5 | 38 | -- | -- | -- | -- | -- | -- | -- | -- | -- | -- | -- |
| b-6 | 52 | -- | -- | -- | -- | -- | -- | -- | -- | -- | -- | -- |
| b-7 | 154 | -- | -- | -- | -- | -- | -- | -- | -- | -- | -- | -- |
| b-8 | 11 | -- | -- | -- | -- | -- | -- | -- | -- | -- | -- | -- |
| b-9 | 142 | -- | -- | -- | -- | -- | -- | -- | -- | -- | -- | -- |
| b-10 | < 20 | -- | -- | -- | -- | -- | -- | -- | -- | -- | -- | -- |
| b-11 | 117 | 10 | -- | -- | -- | -- | -- | -- | -- | -- | -- | -- |
| b-12 | 200 < | -- | 10 | -- | -- | -- | -- | -- | -- | -- | -- | -- |
| b-13 | | -- | -- | -- | -- | -- | -- | -- | -- | -- | -- | -- |
| c-1 | | 5 | 5 | -- | 0.5 | 25 | -- | 5 | 5 | 5 | 5 | 5 |
| c-2 | | -- | -- | -- | -- | -- | -- | -- | -- | -- | -- | -- |
| c-3 | | -- | -- | -- | -- | -- | 5 | -- | -- | -- | -- | -- |
| d-1 | | -- | -- | -- | -- | -- | -- | -- | -- | -- | -- | -- |
| d-2 | | -- | -- | -- | -- | -- | -- | -- | -- | -- | -- | -- |
| d-3 | | 5 | 5 | 5 | 5 | 5 | 5 | -- | 0.5 | 25 | -- | -- |
| d-4 | | -- | -- | -- | -- | -- | -- | -- | -- | -- | 5 | -- |
| d-5 | | -- | -- | -- | -- | -- | -- | -- | -- | -- | -- | 5 |
| e-1 | | 20 | 20 | 20 | 20 | 20 | 20 | 20 | 20 | 20 | 20 | 20 |
| e-2 | | -- | -- | -- | -- | -- | -- | -- | -- | -- | -- | -- |
| e-3 | | -- | -- | -- | -- | -- | -- | -- | -- | -- | -- | -- |
| e-4 | | -- | -- | -- | -- | -- | -- | -- | -- | -- | -- | -- |
| e-5 | | -- | -- | -- | -- | -- | -- | -- | -- | -- | -- | -- |
| e-6 | | -- | -- | -- | -- | -- | -- | -- | -- | -- | -- | -- |
| Carbon | | 5 | 5 | 5 | 5 | 5 | 5 | 5 | 5 | 5 | 5 | 5 |
| f-1 | | -- | -- | -- | -- | -- | -- | -- | -- | -- | -- | -- |
| x-1 | | -- | -- | -- | -- | -- | -- | -- | -- | -- | -- | -- |
| x-2 | | -- | -- | -- | -- | -- | -- | -- | -- | -- | -- | -- |
| Mg element amount | | 1.40 | 1.40 | 1.39 | 1.38 | 0.90 | 1.38 | 1.38 | 1.38 | 0.80 | 1.36 | 1.43 |
| Antibacterial performance | | D | D | D | D | D | D | D | D | D | D | D |

(continued)

| Component | Melting point (°C) | C7 | C8 | C9 | C10 | C11 | C12 | C13 | C14 | C15 | C16 | C17 |
|---|---|---|---|---|---|---|---|---|---|---|---|---|
| Color unevenness | | C | C | C | C | C | C | C | C | C | C | C |
| Impact resistance | | C | C | C | C | C | C | C | C | C | C | C |
| Adhesiveness | | C | C | C | C | C | C | C | C | C | C | C |

[Table 1-6]

| Component | Melting point (°C) | C18 | C19 | C20 | C21 | C22 | C23 | C24 | C25 | C26 | C27 |
|---|---|---|---|---|---|---|---|---|---|---|---|
| a-1 | | 100 | 100 | 100 | -- | -- | 100 | 100 | 100 | 100 | 100 |
| a-2 | | -- | -- | -- | -- | -- | -- | -- | -- | -- | -- |
| a-3 | | -- | -- | -- | -- | -- | -- | -- | -- | -- | -- |
| b-1 | 47 to 54 | 10 | 10 | 10 | -- | -- | 10 | 10 | -- | 10 | 10 |
| b-2 | < 20 | -- | -- | -- | -- | -- | -- | -- | -- | -- | -- |
| b-3 | -60 | -- | -- | -- | -- | -- | -- | -- | -- | -- | -- |
| b-4 | 13 | -- | -- | -- | -- | -- | -- | -- | -- | -- | -- |
| b-5 | 38 | -- | -- | -- | -- | -- | -- | -- | -- | -- | -- |
| b-6 | 52 | -- | -- | -- | -- | -- | -- | -- | -- | -- | -- |
| b-7 | 154 | -- | -- | -- | -- | -- | -- | -- | -- | -- | -- |
| b-8 | 11 | -- | -- | -- | -- | -- | -- | -- | -- | -- | -- |
| b-9 | 142 | -- | -- | -- | -- | -- | -- | -- | -- | -- | -- |
| b-10 | < 20 | -- | -- | -- | -- | -- | -- | -- | -- | -- | -- |
| b-11 | 117 | -- | -- | -- | -- | -- | -- | -- | -- | -- | -- |
| b-12 | 200 < | -- | -- | -- | -- | -- | -- | -- | -- | -- | -- |
| b-13 | | -- | -- | -- | -- | -- | -- | -- | 15 | -- | -- |
| c-1 | | 5 | 5 | 5 | -- | -- | 5 | 5 | 5 | 5 | 5 |
| c-2 | | -- | -- | -- | -- | -- | -- | -- | -- | -- | -- |
| c-3 | | -- | -- | -- | -- | -- | -- | -- | -- | -- | -- |
| d-1 | | -- | -- | -- | -- | -- | -- | -- | -- | -- | -- |
| d-2 | | -- | -- | -- | -- | -- | -- | -- | -- | -- | -- |
| d-3 | | 5 | 5 | 5 | -- | -- | 5 | 5 | 5 | 20 | 2 |
| d-4 | | -- | -- | -- | -- | -- | -- | -- | -- | -- | -- |
| d-5 | | -- | -- | -- | -- | -- | -- | -- | -- | -- | -- |
| e-1 | | -- | 4 | 35 | -- | -- | -- | -- | 20 | 30 | 2 |
| e-2 | | -- | -- | -- | -- | -- | -- | -- | -- | -- | -- |
| e-3 | | -- | -- | -- | -- | -- | -- | -- | -- | -- | -- |
| e-4 | | -- | -- | -- | -- | -- | -- | -- | -- | -- | -- |
| e-5 | | -- | -- | -- | -- | -- | 20 | -- | -- | -- | -- |
| e-6 | | -- | -- | -- | -- | -- | -- | 20 | -- | -- | -- |
| Carbon | | 5 | 5 | 5 | -- | -- | 5 | 5 | 5 | 5 | 5 |

(continued)

| Component | Melting point (°C) | C18 | C19 | C20 | C21 | C22 | C23 | C24 | C25 | C26 | C27 |
|---|---|---|---|---|---|---|---|---|---|---|---|
| f-1 | | -- | -- | -- | -- | -- | -- | -- | -- | -- | -- |
| x-1 | | -- | -- | -- | 100 | -- | -- | -- | -- | -- | -- |
| x-2 | | -- | -- | -- | -- | 100 | -- | -- | -- | -- | -- |
| Mg element amount | | 1.43 | 1.40 | 1.45 | 1.40 | 1.40 | 1.40 | 1.40 | 1.20 | 0.90 | 0.90 |
| Antibacterial performance | | D | D | D | D | D | D | D | D | D | D |
| Color unevenness | | C | C | C | -- | -- | C | C | C | C | C |
| Impact resistance | | C | C | C | C | C | C | C | C | C | C |
| Adhesiveness | | C | C | C | C | C | C | C | C | C | C |

<Note to table>

"E": Example

"C": Comparative Example

"--": with regard to the component, the corresponding component was not contained; regarding the test results of Comparative Examples 21 and 22, since carbon black was not used in Comparative Examples 21 and 22 and the cured adhesive substance was transparent, the test results were not evaluated.

[Component (a)]

a-1:
Bisphenol A diglycidyl ether (trade name "jER828", manufactured by Mitsubishi Chemical Corporation, epoxy equivalent: 189)
a-2:
Bisphenol F diglycidyl ether (trade name "jER807", manufactured by Mitsubishi Chemical Corporation, epoxy equivalent: 170)
a-3:
1,6-Hexanediol diglycidyl ether (manufactured by Tokyo Chemical Industry Co., Ltd.)

[Component (b)]

b-1:
2-Ethyl-4-methylimidazole (melting point: 47°C to 54°C) (manufactured by Sigma-Aldrich Co., LLC)
b-2:
4-Ethylimidazole (liquid at 20°C) (manufactured by Sigma-Aldrich Co., LLC)
b-3:
1-Methylimidazole (melting point: -60°C) (manufactured by Tokyo Chemical Industry Co., Ltd.)
b-4:
1-Phenylimidazole (melting point: 13°C) (manufactured by Sigma-Aldrich Co., LLC)
b-5:
1,2-Dimethylimidazole (melting point: 38°C) (manufactured by Sigma-Aldrich Co., LLC)
b-6:
2-Butylimidazole (melting point: 52°C) (manufactured by Sigma-Aldrich Co., LLC)

The following are imidazole compounds and epoxy resin curing agents used in Comparative Examples.

b-7:
1-(4-Cyanophenyl)imidazole (manufactured by Tokyo Chemical Industry Co., Ltd.)
b-8:
1-Cyanoethyl-2-ethyl-4-methylimidazole (manufactured by Sigma-Aldrich Co., LLC)
b-9:
4(5)-Cyanomethylimidazole (manufactured by Tokyo Chemical Industry Co., Ltd.)
b-10:
1-(3-Aminopropyl)imidazole (manufactured by Tokyo Chemical Industry Co., Ltd.)
b-11:
1-Methylimidazole-2-methanol (manufactured by Sigma-Aldrich Co., LLC)
b-12:
1-Imidazoleacetic acid (manufactured by Tokyo Chemical Industry Co., Ltd.)
b-13:
Methaxylylenediamine (manufactured by Sigma-Aldrich Co., LLC)

[Component (c)]

c-1:
MEH-8000H (trade name, phenol resin which is liquid at 25°C, manufactured by MEIWAKASEI.,LTD.)
c-2:
MEH-8005 (trade name, phenol resin which is liquid at 25°C, manufactured by MEIWAKASEI.,LTD.)

The following is a phenol resin used in Comparative Examples.
c-3:
DL-92 (trade name, solid phenol resin at 25°C, manufactured by MEIWAKASEI.,LTD.)

[Component (d)]

d-1:
AEROSIL RX-200 (trade name, fumed silica surface-treated with hexamethyldisilazane, average particle diameter: 12 nm, manufactured by Evonik Industries AG.)
d-2:
AEROSIL NAX-50 (trade name, fumed silica surface-treated with hexamethyldisilazane, average particle diameter: 30 nm, manufactured by Evonik Industries AG.)
d-3:
AEROSIL RY-200 (trade name, fumed silica surface-treated with dimethyl silicone oil, average particle diameter: 12 nm, manufactured by Evonik Industries AG.)

The following are silica used in Comparative Examples.

d-4:
AEROSIL RX-50 (trade name, fumed silica, average particle diameter: 40 nm, manufactured by Evonik Industries AG.)
d-5:
HPS-3500 (trade name, fused silica, manufactured by Toagosei Co., Ltd.)

[Component (e)]

e-1:
MICRO ACE SG-95 (trade name, talc, average particle diameter: 2.1 μm, manufactured by Nippon Talc Co.,Ltd.)
e-2:
MICRO ACE P-3 (trade name, talc, average particle diameter: 5.0 μm, manufactured by Nippon Talc Co.,Ltd.)

e-3:
MICRO ACE K-1 (trade name, talc, average particle diameter: 8.0 μm, manufactured by Nippon Talc Co.,Ltd.)
e-4:
MICRO ACE MS-P (trade name, talc, average particle diameter: 14.0 μm, manufactured by Nippon Talc Co.,Ltd.)

The following are inorganic fillers used in Comparative Examples.

e-5:
MicronCal MC-120 (trade name, calcium carbonate, average particle diameter: 5.0 μm, ASAHI KOHMATSU CO., LTD.)
e-6:
TP Clay (trade name, clay, average particle diameter: 8.0 μm, manufactured by Sanyou Clay Industry Co., Ltd.)

Carbon:

Carbon black (average particle diameter: 50 nm, manufactured by Mitsubishi Chemical Corporation)

[Component (f)]

f-1:
KBM-403 (trade name, silane coupling agent, manufactured by Shin-Etsu Silicone Co., Ltd.)
The following are adhesives of Comparative Examples.

x-1:
EPO-TEK 353 (trade name, imidazole curing agent-containing epoxy adhesive, manufactured by Epoxy Technology, Inc.)
x-2:
EPO-TEK 354 (trade name, imidazole curing agent-containing epoxy adhesive, manufactured by Epoxy Technology, Inc.)

The adhesives of Comparative Examples 1 and 2 did not meet the specified content of the component (b) according to the present invention. The adhesives of Comparative Examples 3 to 8 used imidazole compounds that did not satisfy the regulation of the present invention. The adhesive of Comparative Example 9 did not contain the component (b). The adhesives of Comparative Examples 10 and 11 did not meet the specified content of the component (c) according to the present invention. The adhesive of Comparative Example 12 used a phenol resin that did not satisfy the regulation of the present invention. The adhesive of Comparative Example 13 did not contain the component (d). The adhesives of Comparative Examples 14 and 15 did not meet the specified content of the component (d) according to the present invention. The adhesive of Comparative Example 16 used fumed silica that did not satisfy the regulation of the present invention. The adhesive of Comparative Example 17 used fused silica without using the component (d). The adhesive of Comparative Example 18 did not contain the component (e). The adhesives of Comparative Examples 19 and 20 did not meet the specified content of the component (e) according to the present invention. The adhesives of Comparative Examples 21 and 22 were commercially available products that did not contain the components (c) to (e). The adhesives of Comparative Examples 23 and 24 used inorganic fillers other than the component (e). The adhesive of Comparative Example 25 used an epoxy resin curing agent other than the component (b). In the adhesives of Comparative Examples 26 and 27, the total of the content of the component (d) and the content of the component (e) did not satisfy the regulations of the present invention. In the medical device members produced using the adhesives of Comparative Examples 1 to 27, the results of Test Example 1 (antibacterial performance), Test Example 3 (impact resistance), and Test Example 4 (adhesiveness) were not sufficient.
On the other hand, the medical device member produced using the adhesive according to the embodiment of the present invention had excellent evaluation results in all of Test Examples 1 to 4 (antibacterial performance, color unevenness, impact resistance, and adhesiveness) (Examples 1 to 38).

Explanation of References

1: substrate
2: cured substance of adhesive

10: medical device member

## Claims

1. An adhesive for a medical device, comprising the following components (a) to (e):

    (a) an epoxy resin including at least one of a bisphenol A-type epoxy resin or a bisphenol F-type epoxy resin;
    (b) an imidazole compound having no heteroatom other than a ring-constituting nitrogen atom and having a melting point of lower than 60°C;
    (c) a phenol resin which is liquid at 25°C;
    (d) fumed silica having an average particle diameter, measured as indicated in the description, of 5 nm or more and 35 nm or less; and
    (e) talc,

    wherein, with respect to 100 parts by mass of a total content of the bisphenol A-type epoxy resin and the bisphenol F-type epoxy resin, a content of the component (b) is 1 to 20 parts by mass, a content of the component (c) is 1 to 20 parts by mass, a content of the component (d) is 1 to 20 parts by mass, a content of the component (e) is 5 to 30 parts by mass, and a total of the content of the component (d) and the content of the component (e) is 6 parts by mass or more and less than 40 parts by mass.

2. The adhesive for a medical device according to claim 1,

    wherein the component (a) includes an epoxy resin represented by General Formula (1),

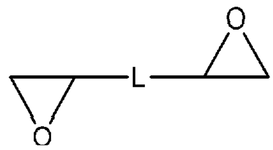

(1)

    in General Formula (1), L represents a linking group formed by combining at least one of an alkylene group, an alkenylene group, or an alkynylene group with an ether bond, an alkylene group, an alkenylene group, or an alkynylene group.

3. The adhesive for a medical device according to claim 1 or 2, further comprising:
    (f) a silane coupling agent.

4. The adhesive for a medical device according to claim 1 or 2,
    wherein a total of the content of the component (b) and the content of the component (c) is 5 parts by mass or more and less than 30 parts by mass with respect to 100 parts by mass of the total content of the bisphenol A-type epoxy resin and the bisphenol F-type epoxy resin.

5. The adhesive for a medical device according to claim 1 or 2,
    wherein a value of a ratio of the content of the component (c) to the content of the component (b) is 0.3 to 1.0.

6. A cured substance obtained by curing the adhesive for a medical device according to claim 1 or 2.

7. A medical device member comprising:

    the cured substance according to claim 6; and
    a substrate.

8. A medical device member comprising:

    a substrate; and
    a sheet which is obtained by curing the adhesive for a medical device according to claim 1 or 2, and is provided on the substrate,

wherein a value of a ratio of a magnesium element amount on a surface of the sheet in contact with the substrate to a magnesium element amount, measured as indicated in the description, on a surface of the sheet on a side opposite to the substrate is 1.0 to 1.3.

**9.** The medical device member according to claim 7,
wherein the substrate is a resin substrate, a glass substrate, or a stainless steel substrate.

**10.** A medical device in which a constituting member is fixed by the cured substance according to claim 6.

**11.** A medical device comprising:
the medical device member according to claim 7.

**12.** The medical device according to claim 10,
wherein the medical device is an endoscope.

**13.** A manufacturing method of a medical device, comprising:
fixing a constituting member using the adhesive for a medical device according to claim 1 or 2.

## Patentansprüche

**1.** Klebstoff für eine medizinische Vorrichtung, umfassend die folgenden Komponenten (a) bis (e):

(a) ein Epoxidharz, das mindestens eines von einem Epoxidharz des Bisphenol A-Typs und einem Epoxidharz des Bisphenol F-Typs enthält;
(b) eine Imidazolverbindung, die kein anderes Heteroatom als ein ringbildendes Stickstoffatom aufweist und einen Schmelzpunkt von niedriger als 60 °C aufweist;
(c) ein Phenolharz, das bei 25 °C flüssig ist;
(d) pyrogenes Siliciumdioxid, das einen durchschnittlichen Teilchendurchmesser, der wie in der Beschreibung angegeben gemessen wird, von 5 nm oder mehr und 35 nm oder weniger aufweist; und
(e) Talk

wobei in Bezug auf 100 Massenteile eines Gesamtgehalts des Epoxidharzes des Bisphenol A-Typs und des Epoxidharzes des Bisphenol F-Typs ein Gehalt der Komponente (b) 1 bis 20 Massenteile, ein Gehalt der Komponente (c) 1 bis 20 Massenteile, ein Gehalt der Komponente (d) 1 bis 20 Massenteile, ein Gehalt der Komponente (e) 5 bis 30 Massenteile beträgt und eine Summe des Gehalts der Komponente (d) und des Gehalts der Komponente (e) 6 Massenteile oder mehr und weniger als 40 Massenteile beträgt.

**2.** Klebstoff für eine medizinische Vorrichtung nach Anspruch 1,

wobei die Komponente (a) ein Epoxidharz, das durch allgemeine Formel (1) dargestellt wird, enthält,

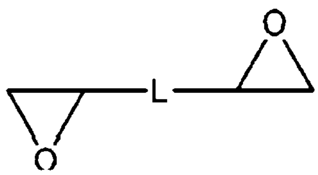

(1)

wobei in allgemeiner Formel (1) L eine Verknüpfungsgruppe darstellt, die durch Kombinieren von mindestens einer von einer Alkylengruppe, einer Alkenylen-Gruppe und einer Alkinylen-Gruppe mit einer Etherbindung, einer Alkylengruppe, einer Alkenylen-Gruppe und einer Alkinylen-Gruppe gebildet wird.

**3.** Klebstoff für eine medizinische Vorrichtung nach Anspruch 1 oder 2, ferner umfassend:
(f) ein Silankupplungsmittel.

**4.** Klebstoff für eine medizinische Vorrichtung nach Anspruch 1 oder 2,
wobei eine Summe des Gehalts der Komponente (b) und des Gehalts der Komponente (c) in Bezug auf 100

Massenteile des Gesamtgehalts des Epoxidharzes des Bisphenol A-Typs und des Epoxidharzes des Bisphenol F-Typs 5 Massenteile oder mehr und weniger als 30 Massenteile beträgt.

**5.** Klebstoff für eine medizinische Vorrichtung nach Anspruch 1 oder 2,
wobei ein Wert eines Verhältnisses des Gehalts der Komponente (c) zu dem Gehalt der Komponente (b) 0,3 bis 1,0 beträgt.

**6.** Ausgehärtete Substanz, die durch Härtung des Klebstoffs für eine medizinische Vorrichtung nach Anspruch 1 oder 2 erhalten wird.

**7.** Medizinisches Vorrichtungselement, umfassend:

die ausgehärtete Substanz nach Anspruch 6; und
ein Substrat;

**8.** Medizinisches Vorrichtungselement, umfassend:

ein Substrat; und
eine Schicht, die durch Härtung des Klebstoffs für eine medizinische Vorrichtung nach Anspruch 1 oder 2 erhalten wird und an dem Substrat vorgesehen ist,
wobei ein Wert eines Verhältnisses einer Magnesiumelementmenge auf einer Oberfläche der Schicht, die mit dem Substrat in Kontakt steht, zu einer Magnesiumelementmenge, die wie in der Beschreibung angegeben gemessen wird, auf einer Oberfläche der Schicht auf einer Seite, die dem Substrat gegenüberliegt, 1,0 bis 1,3 beträgt.

**9.** Medizinisches Vorrichtungselement nach Anspruch 7,
wobei das Substrat ein Harzsubstrat, ein Glassubstrat oder ein Edelstahlsubstrat ist.

**10.** Medizinische Vorrichtung, bei der ein konstituierendes Element durch das ausgehärtete Substanz nach Anspruch 6 befestigt ist.

**11.** Medizinische Vorrichtung, umfassend:
das medizinische Vorrichtungselement nach Anspruch 7.

**12.** Medizinische Vorrichtung nach Anspruch 10,
wobei die medizinische Vorrichtung ein Endoskop ist.

**13.** Herstellungsverfahren einer medizinischen Vorrichtung, umfassend:
Befestigen eines konstituierenden Elements unter Verwendung des Klebstoffs für eine medizinische Vorrichtung nach Anspruch 1 oder 2.

## Revendications

**1.** Adhésif pour un dispositif médical, comprenant les composants suivants (a) à (e) :

(a) une résine époxy incluant au moins l'une d'une résine époxy de type bisphénol A ou d'une résine époxy de type bisphénol F ;
(b) un composé imidazole n'ayant pas d'hétéroatome autre qu'un atome d'azote constitutif de cycle et ayant un point de fusion inférieur à 60 °C ;
(c) une résine phénolique qui est liquide à 25 °C ;
(d) de la silice pyrogénée ayant un diamètre moyen de particules, mesuré comme indiqué dans la description, de 5 nm ou plus et de 35 nm ou moins ; et
(e) du talc,

dans lequel, par rapport à 100 parties en masse d'une teneur totale de la résine époxy de type bisphénol A et de la résine époxy de type bisphénol F, une teneur du composant (b) est de 1 à 20 parties en masse, une teneur du composant (c) est de 1 à 20 parties en masse, une teneur du composant (d) est de 1 à 20 parties en masse, une teneur

du composant (e) est de 5 à 30 parties en masse, et un total d'une teneur du composant (d) et d'une teneur du composant (e) est de 6 parties en masse ou plus et inférieur à 40 parties en masse.

**2.** Adhésif pour un dispositif médical selon la revendication 1,

dans lequel le composant (a) inclut une résine époxy représentée par Formule générale (1),

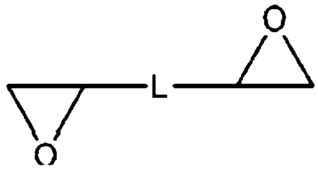

(1)

dans Formule générale (1), L représente un groupe de liaison formé en combinant au moins l'un d'un groupe alkylène, d'un groupe alcénylène ou d'un groupe alkynylène avec une liaison éther, un groupe alkylène, un groupe alcénylène ou un groupe alkynylène.

**3.** Adhésif pour un dispositif médical selon la revendication 1 ou la revendication 2, comprenant en outre : (f) un agent de couplage silane.

**4.** Adhésif pour un dispositif médical selon la revendication 1 ou la revendication 2, dans lequel un total d'une teneur du composant (b) et d'une teneur du composant (c) est de 5 parties en masse ou plus et inférieur à 30 parties en masse par rapport à 100 parties en masse d'une teneur totale de la résine époxy de type bisphénol A et de la résine époxy de type bisphénol F.

**5.** Adhésif pour un dispositif médical selon la revendication 1 ou la revendication 2, dans lequel une valeur d'un rapport d'une teneur du composant (c) à une teneur du composant (b) est de 0,3 à 1,0.

**6.** Substance durcie obtenue en durcissant l'adhésif pour un dispositif médical selon la revendication 1 ou la revendication 2.

**7.** Élément de dispositif médical comprenant :

la substance durcie selon la revendication 6 ; et
un substrat ;

**8.** Élément de dispositif médical comprenant :

un substrat ; et
une feuille qui est obtenue en durcissant l'adhésif pour un dispositif médical selon la revendication 1 ou la revendication 2, et est disposée sur le substrat,
dans laquelle une valeur d'un rapport d'une quantité d'élément de magnésium sur une surface de la feuille en contact avec le substrat à une quantité d'élément de magnésium, mesurée comme indiqué dans la description, sur une surface de la feuille sur un côté opposé au substrat est de 1,0 à 1,3.

**9.** Élément de dispositif médical selon la revendication 7, dans lequel le substrat est un substrat en résine, un substrat en verre ou un substrat en acier inoxydable.

**10.** Dispositif médical dans lequel un élément constitutif est fixé par la substance durcie selon la revendication 6.

**11.** Dispositif médical comprenant : l'élément de dispositif médical selon la revendication 7.

**12.** Dispositif médical selon la revendication 10, dans lequel le dispositif médical est un endoscope.

**13.** Procédé de fabrication d'un dispositif médical, comprenant :

fixer un élément constitutif en utilisant l'adhésif pour un dispositif médical selon la revendication 1 ou la revendication 2.

FIG. 1

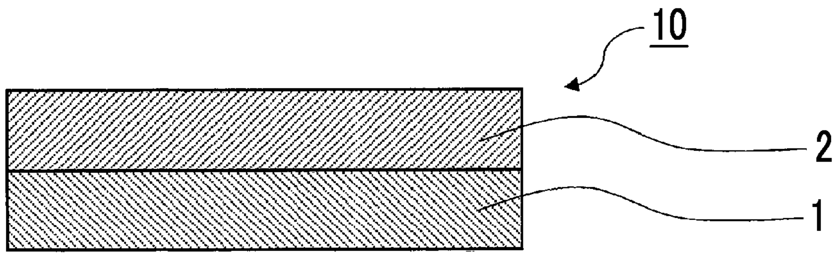

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019041873 A
- JP 2022047458 A
- JP 2007197716 A
- WO 2020175272 A
- JP 2009194934 A
- JP 2014511191 A
- JP 2001128929 A